# EUROPEAN PATENT SPECIFICATION

(11) **EP 2 595 627 B1**
(45) Date of publication and mention of the grant of the patent: **25.03.2015**
(21) Application number: 10737300.3
(22) Date of filing: 22.06.2010
(51) Int. Cl.: A61K 31/438, A61K 31/513, A61K 31/551, A61P 31/00, A61P 31/18, A61K 9/16, A61K 31/475, A61K 36/24

(54) **USE OF ULEINE FOR THE PREVENTION AND/OR THE TREATMENT OF AIDS**
VERWENDUNG VON ULEIN ZUR PRÄVENTION UND/ODER BEHANDLUNG VON AIDS
UTILISATION DE L'ULÉINE POUR LA PRÉVENTION ET/OU LE TRAITEMENT DU SIDA

(43) Date of publication of application: 29.05.2013
(73) Proprietor: Parabolic Biologicals Sprl, 1320 Beauvechain (BE)
(72) Inventor: MAES, Dominique, B-1320 Beauvechain (BE); MAES, Roland, F-67190 Rosheim (FR)
(74) Representative: Pronovem
(86) International application number: PCT/EP2010/058847
(87) International publication number: WO 2011/160684

(56) References cited:
- NARDIN J M ET AL: "Effects of Himatanthus lancifolius on human leukocyte chemotaxis and their adhesion to integrins", PLANTA MEDICA 200808 DE LNKD- DOI:10.1055/S-2008-1074582, vol. 74, no. 10, August 2008 (2008-08), pages 1253-1258, XP002625723, ISSN: 0032-0943
- NARDIN JEANINE MARIE ET AL: "The uleine-rich fraction of Himatanthus lancifolius blocks proliferative responses of human lymphoid cells.", PLANTA MEDICA MAY 2010 LNKD- PUBMED:19950051, vol. 76, no. 7, May 2010 (2010-05), pages 697-700, XP002625724, ISSN: 1439-0221
- DE LIMA M P ET AL: "Alkaloid-rich fraction of Himatanthus lancifolius contains anti-tumor agents against leukemic cells", BRAZILIAN JOURNAL OF PHARMACEUTICAL SCIENCES 2010 FACULDADE DE CIENCIAS FARMACEUTICAS (BIBLIOTECA) BRA LNKD- DOI:10.1590/S1984-82502010000200014, vol. 46, no. 2, April 2010 (2010-04), pages 273-280, XP002625725, ISSN: 1984-8250
- SOUZA W M ET AL: "Antimicrobial activity of alkaloidal fraction from barks of Himatanthus lancifolius", FITOTERAPIA, IDB HOLDING, MILAN, IT, vol. 75, no. 7-8, 1 December 2004 (2004-12-01), pages 750-753, XP004658837, ISSN: 0367-326X, DOI: 10.1016/J.FITOTE.2004.09.012

## Description

### Field of the invention

The present invention is in the field of therapy of infectious diseases, especially AIDS. The present invention is related to the use of an alkaloid (uleine) or a plant extract comprising this alkaloid (uleine), for the prevention and/or the treatment of these infectious diseases, especially AIDS.

### Background of the invention

Many infectious diseases are still not adequately controlled or cured by the drugs and therapies currently available.

The contemporary emergence of epidemics of influenza and the persistence throughout the world of chronic diseases, such as AIDS, tuberculosis and some cancers indicate that the development of effective means of combat against these ailments is not at an end. Vaccines and specific drugs are designed to inactivate the etiologic agents of these ailments and disregard their effect on the immune resources of the treated individual. Collateral damages inflicted by these classic potent treatments, essentially chemotherapy, radiotherapy and surgery, to which one may add the adjuvant (aluminum, mercury, saponin, mineral oils, squalene) of intrinsically weak vaccines, are often severe and may also weaken the immune system of the patients under treatment.

It is now recognized that the nature of the food consumed by the patient may have a significant positive outcome on the issue of infectious diseases and cancer, but the nature of the micro constituents delivered by the food that induce these beneficial effects has not been yet identified.

Furthermore, the reluctance to exploit molecules present in natural products is not justified as five molecules useful to combat cancer are derived from plants: taxol (*Taxus brevifolia*), taxotera (*Taxus baccata*), irinotecan (*Camptoteca acuminata*), vincristin (*Catharantus roseus*) and etoposid (*Podophyllum peltatum*)*.*

It is also known that fruits and vegetables consumption markedly influences the latency of some cancers. The hypothesis that this beneficial effect of fruits and vegetables was due to anti-oxidants has been challenged (Bull. Cancer 2002, 89(3): 293-312). The nature of the beneficial micro-constituents and their mode of action has not yet been pinpointed.

Nutrition greatly helps in the maintenance of the hormonal and immune homeostasis, and the populations of some countries demonstrate an unexpected resilience to some afflictions, which is explained by their diet. This epidemiological proof of the usefulness of some plants as preventive means to maintain the immunological homeostasis of the organism has not yet been fully integrated by modern medicine.

Uleine ((M)Dasycarpidan,1-methylene-) i s present in the bark of various species of plants such as *Himatanthus lancifolius, Aspidosperma ulei, Aspidosperma subincanum* and *Plumeria lancifolia.* The bark of some of these plants is used in traditional medicine as febrifuge, ulcers, marsh fever (paludism), asthma, syphilis, and as a stimulant of contractions of the uterus. One may suppose that different biochemical constituents of the bark induce this variety of effects and also that one or more of these molecules possess by themselves a broad spectrum of activities. The two possibilities are not mutually exclusive.

The molecule uleine ((M)Dasycarpidan,1-methylene-) has been synthesized as well as some derivates of it, but it never found an application in either human or veterinary medicine.

### Aims of the invention

The present invention aims to provide a compound or a plant extract comprising this compound that may be used for improving the prevention and/or the treatment of infectious diseases especially AIDS, including the symptoms of these diseases in a mammal subject, especially in a human patient suffering of one or more of these diseases.

Another aim of the invention is to provide such compound or plant extract that presents no toxicity or low toxicity and could be used as a food additive, possibly in complement with (addition to) known anti-infectious compounds, especially known anti-HIV compounds, used in Highly Active Antiretroviral Therapy (HAART).

### Summary of the invention

The present invention is related to uleine, a plant extract comprising uleine or a composition comprising uleine for use in the prevention and/or the treatment of AIDS induced by retroviruses (HIV-1 and/or HIV 2)in a mammal subject.

In the use according to the invention, the uleine plant extract or the composition is used in combination with one or more anti-HIV compound(s) of Highly Active Antiretroviral Therapy (HAART), preferably a combination of stavudine, lamivudine and nevirapine.

Preferably, in the use according to the invention, the uleine content
is between 10 mg and 200 mg,
is preferably substantially free of ellipticine or the ellipticin content is below 5% (w_{ellipticin}:wᵤₗₑᵢₙₑ).

In the present invention, the mammal subject is preferably a human patient and the uleine or the plant extract is coated on microcrystalline beads of pharmaceutically compatible solid support, preferably coated on microcrystalline beads of cellulose.

Preferred anti-HIV compounds, possibly present in this dual composition are:
Nucleoside and nucleotide Reverse Transcriptase Inhibitors (NRTI),
Non nucleoside Reverse Transcriptase Inhibitors (NNRTI), Protease Inhibitors (PIs),
Integrase Inhibitors (such as Raltegravir),
Entry Inhibitors (or fusion Inhibitors), such as Maraviral and enfuvirtide,
Maturation Inhibitors, such as bevirimat and vivecon, Antiviral Hyperactivation Limiting Therapeutics, virostatics (AV-HALTs),
or a mixture thereof.

Preferred combinations of such anti-HIV compounds are:
combination of Efavirenz and Zidovudine,
combination of efavirenz, tenofovir and emtricitabine,
combination of lopinavir, ritovir, zidovudine and lamivudine or
combination of lopinavir, ritovir, tenofovir and entricitabine or
the most preferred combination of stavudine, lamivudine and nevirapine.

These compounds can be administrated simultaneously or sequentially and can be present in adequate dosages and suitable galenic or food formulation selected by the person skilled in the art.

By substantially free of ellipticine, it is meant that less than about 5%, preferably less than 2%, more preferably less than 1% of ellipticine is present (in the composition or plant extrct); these percentages being expressed as weight percentages (w_{ellipticine}:wᵤₗₑᵢₙₑ).

### Short description of the drawings

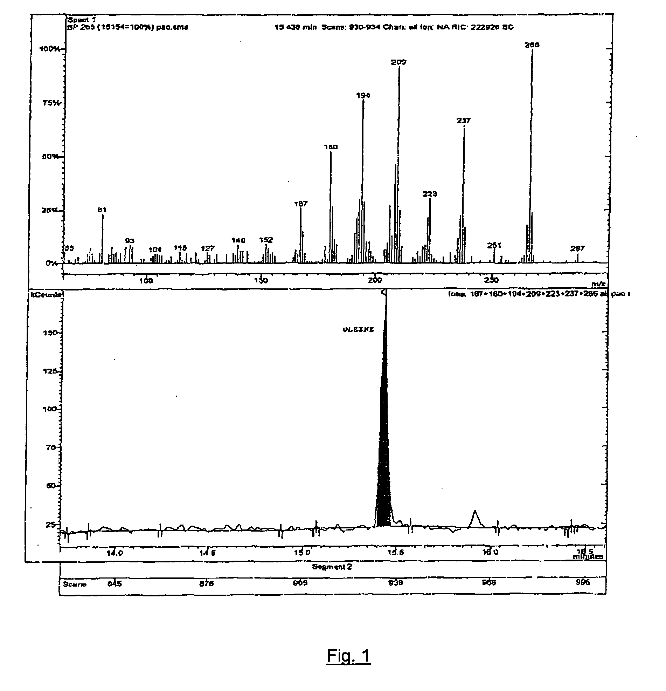

Fig. 1 represents a chromatogram of an extract of *Aspidosperma subincanum* ("pao") and of an uleine ((M)Dasycarpidan,1-methylene-) standard.
Fig. 2 represents the analysis of beads ("granule") coated with a purified extract enriched in uleine.

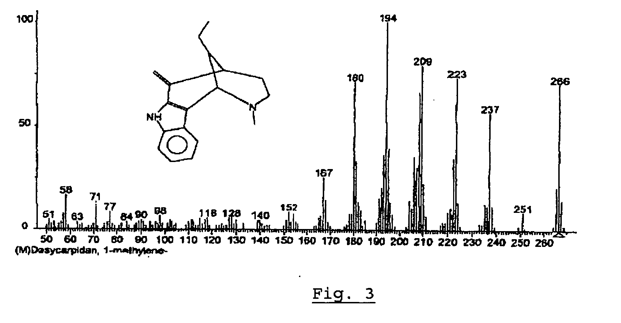

Fig. 3 represents the mass spectrum of the uleine isolated peak.

### Detailed description of the invention

Uleine was found to be the active principle present in a food supplement prepared from the bark of the plant *Aspidosperma subinanum.* This food supplement rich in uleine was shown to improve the physical condition of AIDS patients in a curative way, whose symptoms of the disease as well as those of associated opportunist diseases, including those of a cancerous nature, significantly improved during the intake of the supplement.

Uleine was isolated from *Aspidosperma subincanum* and analyzed the activity and toxicity of this purified substance in healthy animals.

### Presence of uleine in the bark of Aspidosperma subincanum.

A chromatogram of a whole extract of the plant (here named according to one of its indigenous denominations "pao") obtained by High Pressure Liquid Chromatography (HPLC) is given in figure one, together with the chromatogram of an uleine ((M)Dasycarpidan,1-methylene-) standard.

*Isolation of uleine.* Uleine and derivates thereof have been frequently synthesized by chemical means and the isolation methods have been published on several occasions but these syntheses appear to have been exercises serving solely academic purposes.

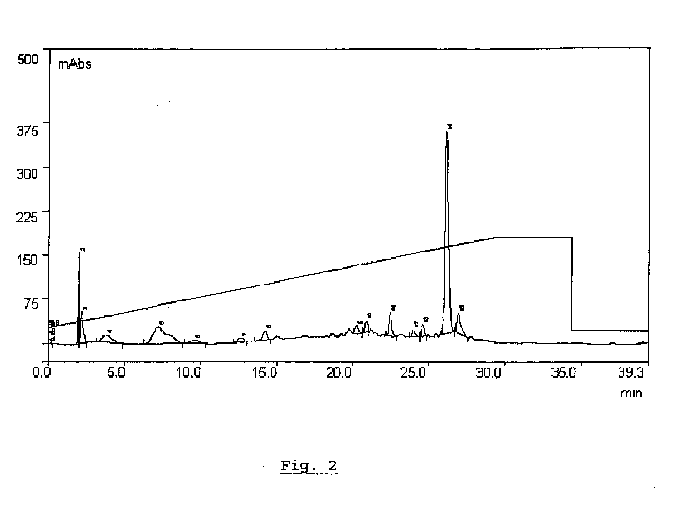

After having ascertained the presence of uleine in an ethanol extract of the bark of *Aspidosperma subincanum,* this extract was further fractionated by HPLC to isolate the uleine peak. This uleine was thereafter coated on beads ("granules") of microcrystalline cellulose and packaged as a food supplement. The purity of the material coated on the beads was verified as shown in figure 2.

The only alkaloid contaminating the preparation that is known to possess an activity (inhibition of cellular growth) is ellipticine. This alkaloid precipitates in saline and acidic media, rendering it improper for either oral absorption, due to the acidity of the stomach, or intra-venous injection due to the salinity of the blood. After intra-venous inoculation of the more soluble acetate of ellipticine, 84% of this substance of improved solubility in saline was excreted in the feces and not in the urine, indicating that this product also is, despite the intra-venous route of administration, rapidly eliminated by fecal excretion. It contaminates the uleine extract object of this application at a concentration less than 0.65% of total alkaloids and its presence at such a low concentration in a food supplement adsorbed orally has been found harmless, as shown by the following toxicity studies conducted with the uleine-enriched extract in healthy animals and diseased patients.

*Activity and toxicity of uleine in healthy animals.* The uleine was coated on microcrystalline beads of cellulose and the beads introduced in caps at concentrations corresponding to 20 mg, 40 mg and 80 mg of total alkaloids being mainly uleine, per cap. Acute and chronic toxicity of these extracts of *Aspidosperma subincanum* enriched in uleine was studied after orally administration to rabbits, rats and mice. A histological examination of various organs obtained from treated rats and rabbits showed neither activity nor toxicity, nor mutagenic or clastogenic activity in the ovaries and testicles, nor in any other organ of these two animal species. No alterations of the germinal cells, in both animal species, could be observed. The materials analyzed behaved as placebos.

Raquel GOLONI, et al. studied in 2005 the acute toxicity of an ethanol extract of Aspidosperma subincanum. (Revista Eletrônica de Farmácia, Suplemento Vol 2 (2), 89-91, 2005: Estudo da Toxicidade Aguda do Aspidosperma Subincanum Martius). A whole ethanol extract of the bark dissolved in saline and dimethyl sulfoxide (DMSO), administered intra peritoneally or orally at a maximal dose of 5000 mg/kg, was without any effect on the survival of the treated animals. This mode of extraction (pure ethanol) and of dissolution (DMSO) would include contaminants as ellipticine within the applied alkaloid extract, while the intraperitoneal route of administration would have authorized the expression of their harmful activity. No acute toxicity was observed, even at doses of 5000 mg/kg administered intraperitoneally.

A group of AIDS adolescents was given the pharmaceutical substance according to the present invention, which comprises an (alkaloid) extract rich in ulein and substantially free of ellipticin under the form of a food supplement during a whole year. A decrease of body weight and a decline of the number of circulating lymphocytes are recognized markers of this pathology. During treatment, symptoms were followed as well as their weight gain, very low at the beginning of the treatment, and the number of their circulating lymphocytes, that was very low at the beginning of the treatment. Within three months following the beginning of the treatment, the body weight and number of lymphocyte markers of the patients taking the food supplement rose spectacularly. The rise in the number of circulating lymphocytes, and not a decline, disproves the possibility of a clastogenic effect of the food supplement orally administered. The opportunist infections associated to their primary AIDS-illness also subdued, including those of a tumor nature (sarcoma and adenopathy) while secondary effects of the treatment remained very rare and of a benign nature (nose bleeding).

One list patients name, body weight before treatment and at different times of treatment, viral load a year before treatment (T-1), lymphocyte number, CD4 number and opportunist infections of which the patients suffered at the moment the treatment was initiated (T0). The patients are subdivided among those under tri therapy (HAART) and those without chemical treatment.

In the vast majority of the cases, under tri therapy or not, with additional natural treatments or not, a recovery is discernable after 3 months of treatment with the uleine-containing extract, and a reduction of the opportunist infections. These infections are listed and ranged from congenital, adenopathy, diarrhea, dermatitis, pneumopathy, hyperthermy, inappetence, otitis, pharingitis, sarcoma, etc. One should note that no spontaneous abortions were reported for the women under study.

Diagnosis of patients is done at their moment of entry (T-1) with a measurement of their viral load and an evaluation of their CD4 lymphocytes. Those in greatest need are treated with anti-virals (Triomine: stavudine, lamivudine & nevirapine) while the others remain untreated.

Three food supplements were given to them about one year after their admission, from time zero (T0) on: Pao pereira Aspidosperma extract at 80 mg of alkaloid (being mainly uleine) at 20 mg of alkaloid being mainly uleine, 2Leid which is composed of nucleic acids and of infinitesimal amounts of cytokines, and Para Immuno, an immunological stimulant composed of propolis, pollen and royal jelly. These supplements, given either alone or together, were administered during twelve months. From the time, the nutrients were given (T0), only a few patients were examined for their CD4 status. The measurement of weight and total number of lymphocytes was made three months after the beginning of the cure (T3) and twelve months later (T12) to assess the effect of the products on the general physical state of the treated patients.

The table I presents results of 14 patients who were under conventional tri-therapy for about a year before addition of the food supplements to the specific treatment. The table II is made of 46 AIDS people who were without specific medical treatment. Three very ill patients died either at the beginning of the treatment or after three months of treatment

### Analyses of the results

### AIDS PATIENTS UNDER SPECIFIC TREATMENT

The first 14 patients (Table I) were under tri-therapy (HAART) during the time comprised between T-1 and T0 in the table. They were given food supplements according to the medical imperatives defined by the treating physician from time T0 to time T12, i.e. during 12 months.

### P.P Aspidosperma 80 + 2Leid + P.I. and tri-therapy (HAART)

The first four patients (n° 1 to n° 4) were in a critical physical condition at T0. They received Pao Aspidosperma extract at 80 units per day throughout the treatment, the cytokines and nucleic acids of 2Leid and the immunostimulant P.I. based on royal jelly, pollen and propolis, in addition to the tri-therapy (HAART) that was pursued.

Patient n° 1 was a 19-year old girl. She weighed 30 kilos at the beginning of the treatment (T0) and the number of her CD4 had collapsed down. Evidently, she did not benefit from the tri-therapy and seemed doomed. In three months of food supplements intake (T3), she gained 15 kilos (from 30 to 45 kilos) and the number of her total lymphocytes increased. She was in good health at T12, after 12 months of the additional alternative treatment.

The tri-therapy followed from time T-1 to T0 appeared more beneficial to patients n° 2, 3 and 4 because they maintained or even slightly increased in weight during that time and maintained their CD4's. The food supplements caused a more dramatic gain in weight, with at least a doubling of its rate. In three months, they gained twelve (from 50 to 62), eleven (from 51 to 62) and ten kilos (from 62 to 72 kilos). More importantly, there was a spectacular increase in their number of total lymphocytes. All were in a satisfactory state of health after 12 months of food supplements intake.

### P.P Aspidosperma .80 + 2Leid and tri-therapy(HAART)

Patients n° 5 to 8 did not benefit from P.I. but received Pao extract as well as cytokines and nucleic acids (2Leid) in addition to the three pharmaceuticals. They were in a very poor state of health at T0: severe drops in CD4 counts, except in patient 8, low weight and low lymphocyte count. Patient n° 5 had her CD4 collapse during tri-therapy. Patients n° 6 and 7 also saw a severe drop in CD4 during tri-therapy, which was a very bad prognostic of survival. After 3 months of food supplement intake, lymphocyte counts increased dramatically and 3 out of 4 patients had a significant gain in weight. Patient 6 also gained weight after 12 months of alternative therapy and all were in a satisfactory state of health at that point (T12).

### P.P.80 and tri-therapy (HAART)

Patients n° 9 to 12 only received P.P.80 as food supplement in addition to the tri-therapy. Patients n° 9 and 10, treated from T0 on according to this regimen, were clinically speaking in a "stable state". Their CD4's were quite high. The weight of patient 9 was not measured before the start of the study, but patient 10 had a stable weight. By contrast, patients n° 11 and 12 had low CD4 counts despite tri therapy and they had lost weight before their enrollment in the study (T-1 to TO), going down from 46 to 40 kilos and from 55 to 50 kilos. Three months after they started taking the P.P. 80 food supplement in addition to tri-therapy, they restored their weight very fast as well as their number of total lymphocytes. After twelve months of treatment with the food supplement, their health was good.

### P.P.Aspidosperma 20 + P.I. or else 2Leid and tri-therapy (HAART)

Patient n° 13, a 15-years old girl weighing 24 kilos at the time of her admission in the orphans' home, under chemotherapy but with diarrhea, fever and pneumopathy at time T0 and having lost ¾ of her CD4's during treatment, recovered when P.P.20 and P.I. was given during 3 months. The use of P.P.20 instead of P.P.80, a 4-fold lower dose in active principles, resulted in a similar weight gain and lymphocyte count recovery as with the previous 12 patients, indicating that this lower dosage was just as efficacious, at least in a teenager.

Patient n° 14 under chemotherapy and receiving 2Leid only as food supplement had a moderate cell count but had lost 5 kilos before joining the study. Three months later, she had not only recovered but gained weight and her lymphocyte count had doubled. These results are comparable to those observed in the other 13 patients, even though this patients' CD4 production before the study and lymphocyte production at the beginning of the study were not completely depressed as in other cases. The results with this patient and patient 13 indicate that different immunostimulants can be used successfully as an adjunct to tri-therapy, even when given alone.

### AIDS PATIENTS WITHOUT SPECIFIC CHEMICAL TREATMENT

The AIDS people presented from n° 15 to 60 did not receive conventional chemotherapy. These are listed in Table 2. The clinicians defined at time T0 their health status following qualitative criteria (conserved, good, bad etc.) and took note of opportunist infections. The loss in weight of these AIDS people from the moment of their admission in the orphans' home (T-1) to time zero (T0) with the implementation of food supplements, is an objective sign of their evolution, as is their viral load and the level of their CD4. Paradoxically, some of them, especially young children, gained weight and increased their numbers of CD4 in the absence of chemical tri-therapy while others saw a decline in their CD4 between T-1 and T0, but only two of them (n° 29 and 41) had CD4 levels below the 100 mark, at time T0.

The table II shows that, irrespective of the severity of their illness and the worsening of their physical condition in the course of time from T-1 to T0, and independently of the given treatment, the AIDS patients taking these food supplements all recovered and gained weight in addition to increasing their total lymphocyte count. All were in satisfactory or good health at the end of the twelve-month course of treatment. If the effects of this treatment are visible after three months in most people, some however needed more than three months of food supplement intake before showing clear evidence of recovering health (cf. Nbs 6, 9, 21, 27, 56) even if an increase in numbers of total lymphocytes took already place at time T3. Note that the physician gave the lower dose of P.P Aspidosperma 20 extract to children and infants, with one exception.

### CONCLUSIONS

It is well known that AIDS tri-therapy (HAART) preferably comprised of stavudine, lamivudine and nevirapine is a heavy treatment that often causes secondary effects in the patient and that therefore this therapy can be a factor of health degradation in many treated subjects. This observation of the treating physician and the results of the study indicate that the therapeutic efficacy of tri-therapy (HAART) as applied in Burundi leaves room for improvement. The results indicate that tri-therapy worsens the health of some AIDS patients instead of improving it, while taking food supplements not only does not cause the same negative effects but can help the patients get over those produced by conventional therapy and even cause partial or full recovery of AIDS patients not receiving it.

These results are very promising and lead to the following conclusions:
- The food supplements are active even in the absence of tri-therapy (HAART).
- The food supplements correct the deleterious effects of the tri-therapy (HAART).
- Pao pereira Aspidosperma extract and 2Leid are both active, even when used alone.
- Two patients treated with P.P Aspidosperma.80 extract suffered from nose bleeding, which disappeared after treatment was suspended. However, the collected data indicates that the dosage might be reduced, as the use of P.P.Aspidosperma20 extract caused similar improvements without any secondary effect in one group of patients.
- No other secondary effect or toxicity was observed with these food supplements.

The extract of Pao pereira Aspidosperma is active on the multiplication of cancers and viruses whereas the RNA included in the 2Leid, and also produced by Parabolic biologicals, stimulates the multiplication of lymphocytes. Para Immuno is a general immunopotentiator. An examination of the results indicates that P.P.20 extract to P.P.80 extract, together with RNA, appear to be the choice regimen to obtain a durable improvement of the health of AIDS patients, even in the absence of tri-therapy (HAART).

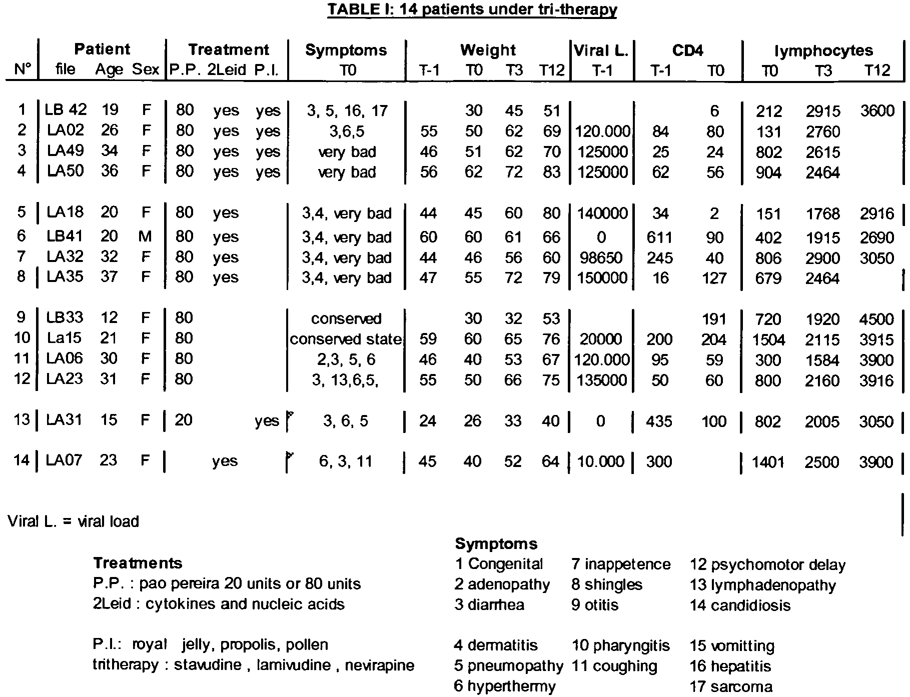

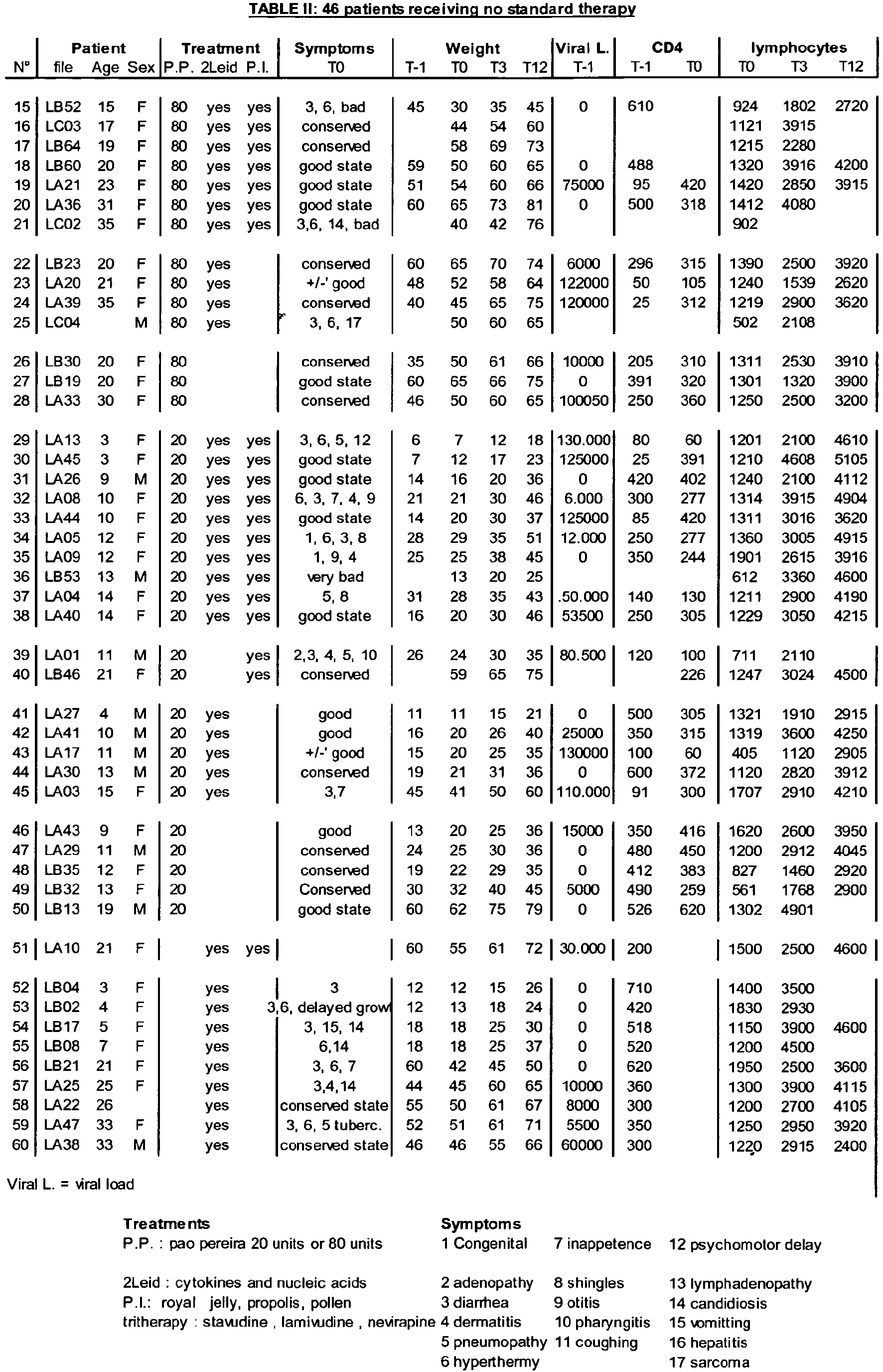

## Claims

1. Uleine, a plant extract comprising uleine or a composition comprising uleine for use in the prevention and/or the treatment of AIDS in a mammal subject.

2. Uleine, a plant extract comprising uleine or a composition comprising uleine for the use according to claim 1, wherein the uleine, the plant extract or the composition is used in combination with one or more anti-HIV compound(s) of Highly Active Antiretroviral Therapy (HAART).

3. Uleine, a plant extract comprising uleine or a composition comprising uleine for the use according to claim 2, wherein the anti-HIV compounds are a combination of stavudine, lamivudine and nevirapine.

4. Uleine, a plant extract comprising uleine or a composition comprising uleine for the use according to any of the preceding claims 1 to 3, wherein the uleine content is between 10 mg to 200 mg.

5. Uleine, a plant extract comprising uleine or a composition comprising uleine for the use according to any of the preceding claims 1 to 4, wherein the plant extract or the composition is substantially free of ellipticine.

6. Uleine, a plant extract comprising uleine or a composition comprising uleine for the use according the claim 5, wherein the ellipticin content in the plant extract or composition is below 5% (w_{ellipticin}:wᵤₗₑᵢₙₑ).

7. Uleine, a plant extract comprising uleine or a composition comprising uleine for the use according to any of the preceding claims 1 to 6, wherein the mammal subject is a human patient.

8. Uleine, a plant extract comprising uleine or a composition comprising uleine for the use according to any of the preceding claims 1 to 7, wherein the uleine or the plant extract is coated on microcrystalline beads of pharmaceutically compatible solid support.

9. Uleine, a plant extract comprising uleine or a composition comprising uleine for the use of claim 8, wherein the uleine or the plant extract is coated on microcrystalline beads of cellulose.

## Patentansprüche

1. Ulein, ein Pflanzenextrakt umfassend Ulein oder eine Zusammensetzung umfassend Ulein zur Verwendung bei der Verhütung und/oder Behandlung von AIDS in einem Säugetier-Probanden.

2. Ulein, ein Pflanzenextrakt, umfassend Ulein oder eine Zusammensetzung umfassend Ulein zur Verwendung nach Anspruch 1, wobei das Ulein, der Pflanzenextrakt oder die Zusammensetzung jeweils in Kombination mit einer oder mehreren Anti-HIV Verbindung(en) der Hoch Aktiven Antiretroviralen Therapie (HAART) eingesetzt werden.

3. Ulein, ein Pflanzenextrakt, umfassend Ulein oder eine Zusammensetzung umfassend Ulein zur Verwendung nach Anspruch 2, wobei die Anti-HIV-Verbindungen eine Kombination aus Stavudin, Lamivudin und Nevirapin sind.

4. Ulein, ein Pflanzenextrakt, umfassend Ulein oder eine Zusammensetzung umfassend Ulein zur Verwendung nach einem der vorstehenden Ansprüche 1 bis 3, wobei der Uleingehalt zwischen 10 mg bis 200 mg beträgt.

5. Ulein, ein Pflanzenextrakt, umfassend Ulein oder eine Zusammensetzung umfassend Ulein zur Verwendung nach einem der vorstehenden Ansprüche 1 bis 4, wobei der Pflanzenextrakt oder die Zusammensetzung im Wesentlichen frei von Ellipticin sind.

6. Ulein, ein Pflanzenextrakt, umfassend Ulein oder eine Zusammensetzung umfassend Ulein zur Verwendung nach Anspruch 5, wobei der Ellipticingehalt im Pflanzenextrakt oder der Zusammensetzung unter 5 % liegt (W_{Ellipticin}:W_{Ulein}).

7. Ulein, ein Pflanzenextrakt, umfassend Ulein oder eine Zusammensetzung umfassend Ulein zur Verwendung nach einem der vorstehenden Ansprüche 1 bis 6, wobei es sich beim Säugetier-Probanden um einen menschlichen Patienten handelt.

8. Ulein, ein Pflanzenextrakt, umfassend Ulein oder eine Zusammensetzung umfassend Ulein zur Verwendung nach einem der vorstehenden Ansprüche 1 bis 7, wobei das Ulein oder der Pflanzenextrakt auf mikrokristallinen Kügelchen eines pharmazeutisch kompatiblen Feststoffträger beschichtet ist.

9. Ulein, ein Pflanzenextrakt, umfassend Ulein oder eine Zusammensetzung umfassend Ulein zur Verwendung nach Anspruch 8, wobei das Ulein oder der Pflanzenextrakt auf mikrokristallinen Kügelchen aus Cellulose beschichtet ist.

## Revendications

1. Uléine, extrait de plante comprenant de l'uléine ou composition comprenant de l'uléine pour l'utilisation dans la prévention et/ou le traitement du SIDA chez un sujet mammifère.

2. Uléine, extrait de plante comprenant de l'uléine ou composition comprenant de l'uléine pour l'utilisation selon la revendication 1, l'uléine, l'extrait de plante ou la composition étant utilisés en association avec un ou plusieurs composés anti-VIH du traitement antirétroviral hautement actif (HAART).

3. Uléine, extrait de plante comprenant de l'uléine ou composition comprenant de l'uléine pour l'utilisation selon la revendication 2, les composés anti-VIH étant une association de stavudine, de lamivudine et de névirapine.

4. Uléine, extrait de plante comprenant de l'uléine ou composition comprenant de l'uléine pour l'utilisation selon l'une quelconque des revendications précédentes 1 à 3, la teneur en uléine étant comprise entre 10 mg et 200 mg.

5. Uléine, extrait de plante comprenant de l'uléine ou composition comprenant de l'uléine pour l'utilisation selon l'une quelconque des revendications précédentes 1 à 4, l'extrait de plante ou la composition étant essentiellement dépourvus d'ellipticine.

6. Uléine, extrait de plante comprenant de l'uléine ou composition comprenant de l'uléine pour l'utilisation selon la revendication 5, la teneur en ellipticine dans l'extrait de plante ou la composition étant inférieure à 5 % (p_{ellipticine}/p_{uléine}).

7. Uléine, extrait de plante comprenant de l'uléine ou composition comprenant de l'uléine pour l'utilisation selon l'une quelconque des revendications précédentes 1 à 6, le sujet mammifère étant un patient humain.

8. Uléine, extrait de plante comprenant de l'uléine ou composition comprenant de l'uléine pour l'utilisation selon l'une quelconque des revendications précédentes 1 à 7, l'uléine ou l'extrait de plante étant appliqué sur des perles microcristallines constituées d'un support solide pharmaceutiquement compatible.

9. Uléine, extrait de plante comprenant de l'uléine ou composition comprenant de l'uléine pour l'utilisation selon la revendication 8, l'uléine ou l'extrait de plante étant appliqué sur des perles microcristallines constituées de cellulose.
